## Europäisches Patentamt

## European Patent Office

## Office européen des brevets

(19)

(11) Veröffentlichungsnummer: **0 037 008**
**A1**

(12) **EUROPÄISCHE PATENTANMELDUNG**

(21) Anmeldenummer: **81101991.8**

(22) Anmeldetag: **17.03.81**

(51) Int. Cl.³: **A 61 B 6/02**

(30) Priorität: **20.03.80 DE 3010780**

(43) Veröffentlichungstag der Anmeldung:
**07.10.81 Patentblatt 81/40**

(84) Benannte Vertragsstaaten:
**DE FR GB**

(71) Anmelder: **SIEMENS AKTIENGESELLSCHAFT Berlin und München**
**Postfach 22 02 61**
**D-8000 München 22(DE)**

(72) Erfinder: **Pfeiler, Manfred, Dr.**
**Ludwig-Thoma-Strasse 25**
**D-8520 Erlangen(DE)**

(72) Erfinder: **Tschunt, Edgar**
**Im Winkel 9**
**D-8521 Rathsberg(DE)**

(54) **Strahlendiagnostikeinrichtung.**

(57) Die Erfindung bezieht sich auf eine Strahlendiagnostikeinrichtung mit einer Patientenliege (6), mit einer Strahlenmeßanordnung (1,4) aus einer Strahlenquelle (1), die mindestens eine das Aufnahmeobjekt (5) durchdringendes, fächerförmiges Strahlenbündel (2) erzeugt, und mit einem Strahlenempfänger (4), der aus einer Reihe von Detektoren besteht, die an einer Signalverarbeitungsschaltung (20) angeschlossen sind, sowie mit Mitteln (16, 17, 18, 19) zur Erzeugung einer Relativbewegung zwischen der Patientenliege (6) und der Strahlenmeßanordnung (1, 4) in Liegenlängsrichtung für die Erzeugung eines Schattenbildes. Es sind Mittel (16, 17) zur Erzeugung mehrerer Strahlenrichtungen vorhanden, so daß sich eine Vielzahl von Kreuzungspunkten der Strahlenwege innerhalb eines Bildaufnahmebereiches ergibt. Die Signalverarbeitungsschaltung (20) bestimmt für jeden Kreuzungspunkt einer parallel zur Patientenliege (6) verlaufenden Ebene (S) die Strahlentransparenz im Patienten (5).

FIG 1

EP 0 037 008 A1

SIEMENS AKTIENGESELLSCHAFT
Berlin und München

Unser Zeichen
VPA 80 P 5035   E

Strahlendiagnostikeinrichtung

Die Erfindung betrifft eine Strahlendiagnostikeinrichtung mit einer Patientenliege, mit einer Strahlenmeßanordnung aus einer Strahlenquelle, die mindestens ein das Aufnahmeobjekt durchdringendes, quer zur Patientenliege verlaufendes, fächerförmiges Strahlenbündel erzeugt, und einem Strahlenempfänger, der aus einer Reihe von Detektoren besteht, die an einer Signalverarbeitungsschaltung angeschlossen sind, sowie mit Mitteln zur Erzeugung einer Relativbewegung zwischen der Patientenliege und der Strahlenmeßanordnung in Liegenlängsrichtung für die Erzeugung eines Schattenbildes aus den Detektor-Ausgangssignalen durch die Signalverarbeitungsschaltung.

Eine Strahlendiagnostikeinrichtung dieser Art ist in der DE-OS 26 13 809 beschrieben. Bei der bekannten Strahlendiagnostikeinrichtung handelt es sich um einen Computertomographen, der mit Mitteln ergänzt ist, die es erlauben, ein Schattenbild eines bestimmten Bereiches eines Patienten anzufertigen. Für die Anfertigung eines Computertomogrammes wird die Meßanordnung um die Liegenlängsachse oder eine dazu parallele Achse gedreht. Aus den Ausgangssignalen der Detektoren berechnet ein Computer dabei die Schwächungswerte von in einer Matrix angeordneten Bildpunkten, die dann als Bild der untersuchten Transversalschicht des Patienten wiedergegeben werden können. Für die Erzeugung eines Schattenbildes wird die Meßanordnung gegen Drehung verriegelt und es erfolgt eine Relativbewegung zwischen der Patientenliege und der

Tp 5 Ler / 6.3.1980

- 2 -     VPA 80 P 5035  E

Meßanordnung in Liegenlängsrichtung. Ein Schattenbild enthält dabei in überlagerter Form alle Details des durchstrahlten Aufnahmebereiches. Da diese Details einander überlagert sind, beispielsweise bei der Betrachtung des Brustkorbes eine Überlagerung der vorderen und der hinteren Rippenteile erfolgt, ist das Bild wenig aussagekräftig.

Zur Verbesserung von Röntgenschattenbildern ist es bereits bekannt, nur eine parallel zur Patientenliege verlaufende Schicht des Patienten dadurch scharf abzubilden, daß man die Röntgenröhre und eine Röntgenfilmkassette fest miteinander koppelt und diese Einheit um eine in der gewünschten Schicht liegende Schwenkachse schwenkt. In diesem Fall werden nur die Details der ausgewählten Schicht auf dem Röntgenfilm scharf abgebildet, während die übrigen Details verwischt abgebildet werden. Nachteilig ist dabei, daß das Sekundärstrahlenraster, das auf den Fokus der Röntgenröhre fokussiert sein muß, wegen der erforderlichen Bewegung der aus Röntgenröhre und Röntgenfilmkassette bestehenden Aufnahmeeinheit nur Lamellen aufweisen kann, die in der Bewegungsrichtung verlaufen. Dadurch ergibt sich eine nur unvollkommene Streustrahlenunterdrückung.

Der Erfindungliegt die Aufgabe zugrunde, ein Strahlendiagnostikgerät der eingangs genannten Art zu schaffen, mit dem es möglich ist, Aufnahmen gewünschter, parallel zur Patientenliege verlaufender Schichten des Patienten anzufertigen, wobei die Auswirkung der Streustrahlung auf die Bildqualität so gering wie möglich gehalten ist.

Diese Aufgabe ist erfindungsgemäß dadurch gelöst, daß Mittel zur Erzeugung mehrerer Strahlenrichtungen vorhanden sind, so daß sich eine Vielzahl von Kreuzungspunkten der Strahlenwege innerhalb eines Bildaufnahmebereiches ergibt und daß die Signalverarbeitungsschaltung so ausgebildet ist, daß sie für jeden Kreuzungspunkt einer parallel zur Patientenliege verlaufenden Ebene die Strahlentransparenz im Patienten bestimmt. Bei der erfindungsgemäßen Strahlendiagnostikeinrichtung kann die Streustrahlung durch einen vor dem Strahlenempfänger angeordneten Kollimator gut unterdrückt werden. Ein Röntgenschattenbild einer zur Patientenliege parallel verlaufenden Schicht kann durch die Signalverarbeitungsschaltung in einfacher Weise berechnet und auf einem Sichtgerät wiedergegeben werden.

Eine Ausführungsform der Erfindung besteht darin, daß die Strahlenquelle mit dem aus einer einzigen Detektorreihe bestehenden Strahlenempfänger starr verbunden und um ihren Fokus schwenkbar ist. In diesem Fall kann die Abtastung des Bildaufnahmebereiches in der Weise erfolgen, daß zunächst eine Schwenkung der Strahlenmeßanordnung um einen vorbestimmten Winkel, dann eine Relativbewegung zwischen Strahlenmeßanordnung und Patientenliege um einen vorbestimmten Schritt, dann wieder eine Schwenkung, dann wieder eine Relativbewegung usw. erfolgt. Eine andere Variante besteht darin, daß die Strahlenquelle mehrere Strahlenfächer unter verschiedenen Richtungen aussendet, von denen jeder von einer Detektorreihe empfangen wird, und daß die Strahlenquelle und alle Detektorreihen ortsfest im Gerät angeordnet sind. In jedem Fall genügt eine Bewegung der Patientenliege zur Abtastung des Bildaufnahmebereiches.

Die Erfindung ist nachfolgend anhand der Zeichnung näher erläutert. Es zeigen:

Fig. 1 und 2 zwei schematische Darstellungen zweier Varianten der Erfindung, und

Fig. 3 und 4 eine Ansicht einer Strahlendiagnostikeinrichtung nach der Erfindung aus
zwei zueinander senkrechten Richtungen.

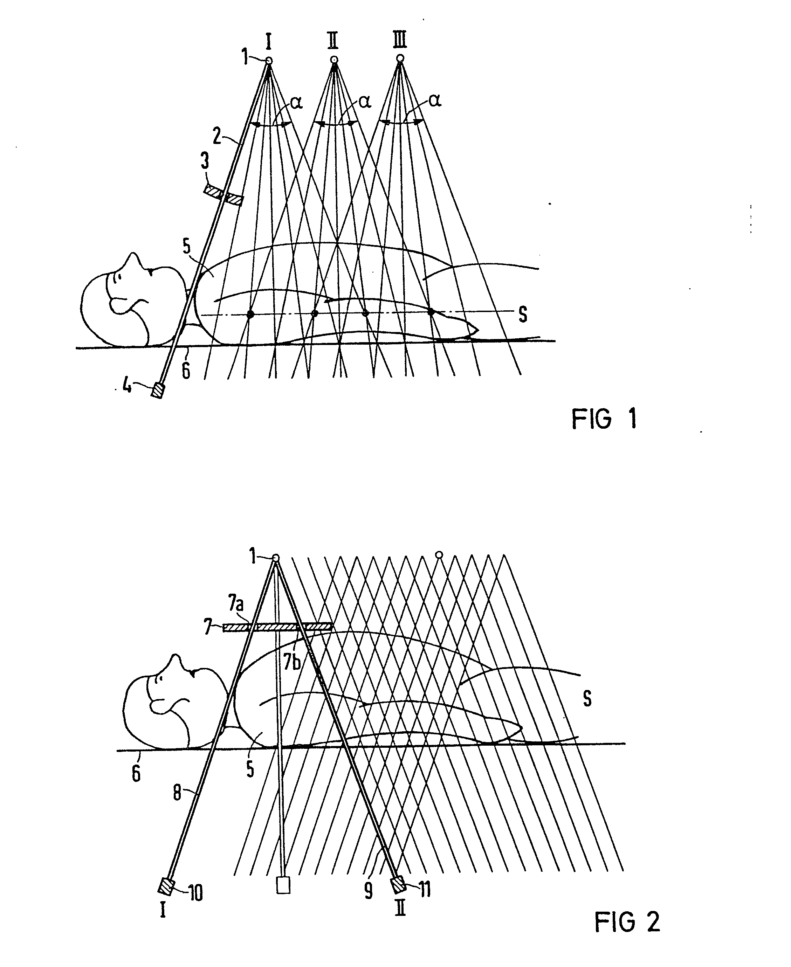

In der Figur 1 ist der Fokus 1 einer Röntgenröhre dargestellt, der ein fächerförmiges Röntgenstrahlenbündel 2
aussendet, das durch eine Primärstrahlenblende 3 eingeblendet wird. Das Röntgenstrahlenbündel 2 wird von einer
Detektorreihe 4 empfangen, die beispielsweise als Ausschnitt aus einem Kreisring ausgebildet und aus 512 Einzeldetektoren bestehen kann. Die Meßanordnung 1, 4 dient
zur Abtastung eines Patienten 5, der auf einer in ihrer
Längsrichtung verschiebbaren Liege 6 ruht.

Die Abtastung des Bildaufnahmebereiches des Patienten 5
erfolgt in der Weise, daß in der gezeichneten Stellung I
des Fokus 1 die Röntgenröhre und der Strahlenempfänger 4
um den Fokus 1 um den Winkel $\alpha$ geschwenkt werden. Während der Schwenkung werden in vorbestimmten Winkelstellungen die Ausgangssignale der Einzeldetektoren des
Strahlenempfängers 4 abgefragt. In der Figur 1 sind sieben Strahlenwege dargestellt, in denen diese Abfrage erfolgt. In der Praxis werden es natürlich wesentlich mehr
Strahlenwege sein. Anschließend an die Schwenkbewegung
erfolgt eine Längsverschiebung der Röntgenröhre und des
starr damit verbundenen Strahlenempfängers 4, bis der
Fokus in der Stellung II liegt. In dieser Stellung erfolgen wieder eine Schwenkung um den Winkel $\alpha$ und eine
Abfrage entsprechend dem vorher beschriebenen Fall. Danach wird die Röntgenröhre mit dem Strahlenempfänger 4
so verschoben, daß der Fokus die Lage III einnimmt. In
dieser Lage erfolgen wieder eine Verschwenkung und eine
Signalabfrage usw. Der Winkel $\alpha$ kann beispielsweise
40$^\circ$ betragen.

Aus der Figur 1 geht hervor, daß die einzelnen Strahlenwege des quer zur Patientenliege 6 verlaufenden Strahlenbündels 2 im Bildaufnahmebereich des Patienten 5 eine Vielzahl von Kreuzungspunkten haben, die sich aus den verschiedenen Strahlenrichtungen ergeben. Durch eine Signalverarbeitungsschaltung, die einen Computer enthält, ist es möglich, für die in einer parallel zur Patientenliege 6 verlaufenden Ebene liegenden Kreuzungspunkte die Strahlentransparenz im Patienten 5 zu bestimmen und bildlich wiederzugeben. Auf diese Weise erhält man ein Röntgenschattenbild einer Längsschicht des Patienten. Der Computer verarbeitet dabei die Ausgangssignale der Einzeldetektoren des Strahlenempfängers 4, die als strahlenelektrische Wandler ausgebildet sind.

In Abwandlung des beschriebenen Abtastverfahrens ist es auch möglich, auf die Längsverschiebung der Strahlenmeßanordnung 1, 4 zu verzichten, wenn sie durch eine Längsverschiebung der Patientenliege 6 mit dem Patienten 5 ersetzt wird. In diesem Fall genügt es, wenn die Meßanordnung 1, 4 um den Fokus 1 schwenkbar gelagert wird.

Bei dem Ausführungsbeispiel gemäß Figur 2 ist eine Primärstrahlenblende 7 vorhanden, die zwei fächerförmige Röntgenstrahlenbündel 8, 9 einblendet, die den Patienten 5 unter verschiedenen Richtungen durchsetzen. Jedes Strahlenbündel 8, 9 wird von je einem Strahlenempfänger 10, 11 empfangen, der jeweils wieder aus einer Reihe von Einzeldetektoren, beispielsweise 512 Einzeldetektoren, besteht. In diesem Fall können die Röntgenröhre sowie die Strahlenempfänger 10, 11 ortsfest im Gerät angeordnet sein. Die Abtastung des Bildaufnahmebereiches kann dadurch erfolgen, daß die Patientenliege 6 mit dem Patienten 5 um ein vorbestimmtes Maß in ihrer Längsrichtung verschoben wird. Auf diese Weise erhält man, wie dies in der Figur 2 dargestellt ist, ebenfalls eine Vielzahl von Kreuzungspunkten, die in der beschriebenen Weise die Grundlage für die Bilderzeugung bilden.

Es ist bei dem Beispiel gemäß Figur 2 möglich, die Blendenöffnungen 7a und 7b so zu steuern, daß sie erst dann geöffnet werden, wenn die Strahlung Signale erzeugt, die auch tatsächlich zur Bilderzeugung benutzbar sind. So kann beispielsweise der Abtastvorgang aus der gezeichneten Stellung heraus in der Weise erfolgen, daß zunächst nur das Röntgenstrahlenbündel 9 vorhanden ist, und erst dann, wenn sich Kreuzungspunkte in dem Patienten 5 ergeben, das Röntgenstrahlenbündel 8 aufgeblendet wird. Am Ende des Vorschubes schließt dann zunächst die Blendenöffnung 7b und erst danach die Blendenöffnung 7a. Dadurch kann die Strahlenbelastung des Patienten 5 klein gehalten werden.

In der Figur 2 sind die Kreuzungspunkte der Strahlenwege dadurch dargestellt, daß der Fokus 1 wandernd und die Patientenliege 6 ortsfest gezeichnet sind. Dies ist zwar eine Möglichkeit zur Abtastung des Bildaufnahmebereiches; wie oben beschrieben, erfolgt jedoch bei dem Beispiel die Relativbewegung in Liegenlängsrichtung dadurch, daß nur die Patientenliege 6 verschoben wird. An der Lage der Kreuzungspunkte ändert sich dadurch nichts.

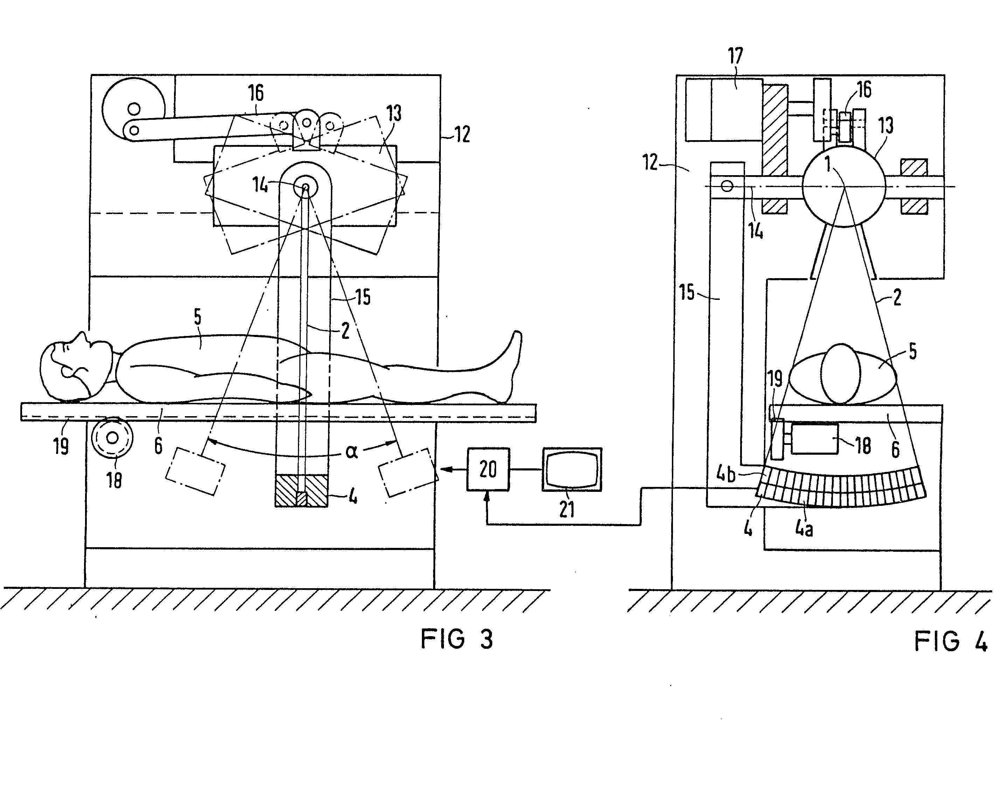

In den Figuren 3 und 4 ist eine Ansicht einer Strahlendiagnostikeinrichtung nach der Erfindung dargestellt, durch die die in Verbindung mit der Figur 1 an zweiter Stelle beschriebene Abtastbewegung erfolgen kann. An einem Geräterahmen 12 ist eine Röntgenröhre 13 um eine horizontale Achse 14 schwenkbar gelagert. Die Röntgenröhre 13 ist über einen Arm 15 mit dem Strahlenempfänger 4 fest verbunden. Aus der Figur 4 geht hervor, daß
der Strahlenempfänger 4 aus einer Reihe 4a von Einzeldetektoren besteht, denen ein Kollimator 4b vorgeschaltet ist.

Über einen Kurbeltrieb 16, der durch einen Motor 17 angetrieben wird, ist die Röntgenröhre 13 um die Achse 14,
die durch den Fokus 1 geht, um den in der Figur 3 dargestellten Winkel $\alpha$ schwenkbar. Der Strahlenempfänger 4
nimmt an dieser Schwenkbewegung teil und kann somit in
die in der Figur 3 gezeichneten Lagen verschwenkt werden.
Die Liegenlängsbewegung erfolgt durch einen Motor 18,
der in eine Zahnstange 19 an der Unterseite der Patientenliege 6 eingreift. Ferner ist ein Computer 20 vorhanden, der am Strahlenempfänger 4 angeschlossen ist und
das jeweilige Röntgenschattenbild der ausgewählten
Schicht berechnet und seine Wiedergabe auf einem Sichtgerät 21 bewirkt. In der Figur 1 ist eine solche mögliche Schicht beispielsweise eingezeichnet und mit S bezeichnet. Für die Berechnung des Bildes dieser Schicht
werden die Kreuzungspunkte herangezogen, die in der
Figur 1 stark markiert sind.

Bei dem Beispiel gemäß Figur 1 ist es auch möglich, nur
die Primärstrahlenblende 3 mit dem aus einer einzigen
Detektorreihe bestehenden Strahlenempfänger 4 starr zu
verbinden und um den Fokus 1 der feststehenden Röntgenröhre schwenkbar anzuordnen.

4 Figuren
4 Patentansprüche

Patentansprüche

1. Strahlendiagnostikeinrichtung mit einer Patientenliege, mit einer Strahlenmeßanordnung aus einer Strahlenquelle, die mindestens ein das Aufnahmeobjekt durchdringendes, quer zur Patientenliege verlaufendes, fächerförmiges Strahlenbündel erzeugt, und einem Strahlenempfänger, der aus einer Reihe von Detektoren besteht, die an einer Signalverarbeitungsschaltung angeschlossen sind, sowie mit Mitteln zur Erzeugung einer Relativbewegung zwischen der Patientenliege und der Strahlenmeßanordnung in Liegenlängsrichtung für die Erzeugung eines Schattenbildes aus den Detektor-Ausgangssignalen durch die Signalverarbeitungsschaltung, d a d u r c h  g e k e n n z e i c h n e t , daß Mittel (16, 17, 18, 19) zur Erzeugung mehrerer Strahlenrichtungen vorhanden sind, so daß sich eine Vielzahl von Kreuzungspunkten der Strahlenwege innerhalb eines Bildaufnahmebereiches ergeben und daß die Signalverarbeitungsschaltung (20) so ausgebildet ist, daß sie für jeden Kreuzungspunkt einer parallel zur Patientenliege (6) verlaufenden Ebene (S) die Strahlentransparenz im Patienten (5) bestimmt.

2. Strahlendiagnostikeinrichtung nach Anspruch 1, d a d u r c h  g e k e n n z e i c h n e t , daß die Strahlenquelle (13) mit dem aus einer einzigen Detektorreihe bestehenden Strahlenempfänger (4) starr verbunden und um ihren Fokus (1) schwenkbar ist.

3. Strahlendiagnostikeinrichtung nach Anspruch 1, d a d u r c h  g e k e n n z e i c h n e t , daß die Strahlenquelle mehrere Strahlenfächer (8, 9) unter verschiedenen Richtungen aussendet, von denen jeder von einer Detektorreihe (10, 11) empfangen wird, und daß die Strahlenquelle und alle Detektorreihen (10, 11) ortsfest im Gerät angeordnet sind.

4. Strahlendiagnostikeinrichtung nach Anspruch 1, d a d u r c h   g e k e n n z e i c h n e t ,   daß nur die Primärstrahlenblende (3) mit dem aus einer einzigen Detektorreihe bestehenden Strahlenempfänger (4) starr verbunden und um den feststehenden Fokus (1) schwenkbar ist.

2/1

FIG 1

FIG 2

FIG 3

FIG 4

2/2

0037008

**Europäisches Patentamt**

# EUROPÄISCHER RECHERCHENBERICHT

0037008

Nummer der Anmeldung

EP 81 10 1991

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 4 179 100 (D. SASHIN et al./UNIVERSITY OF PITTSBURGH)<br><br>* Zusammenfassung; Spalte 8, Zeilen 11-40; Spalte 11, Zeile. 37 - Spalte 12, Zeile 45 und Abbildungen 3a,11,12b *<br><br>-- | 1,2 |
| A | FR - A - 2 345 983 (SIEMENS A.G.)<br><br>* Seite 1, Zeile 35 - Seite 4, Zeile 34; Seite 5, Zeilen 20-34; und Abbildungen 1,2 * | 1,2 |
| D | & DE - A - 2 613 809<br><br>-- | |
| | FR - A - 2 380 593 (TOKYO SHIBAURA ELECTRIC CO. LTD.)<br><br>* Seite 2, Zeile 5 - Seite 3, Zeile 19; Seite 4, Zeile 38 - Seite 7, Zeile 2; Abbildungen 1,2 *<br><br>-- | 1,2 |
| | FR - A - 2 260 977 (DIGITAL INFORMATION SCIENCE CORP.)<br><br>* Seite 2, Zeilen 11-30; Seite 3, Zeile 25 - Seite 4, Zeile 20; Seite 8, Zeile 17 - Seite 9, Zeile 3; Abbildungen 1,4,5 *<br><br>-- | 1 |
| | GB - A - 2 028 053 (SIEMENS A.G.)<br><br>* Zusammenfassung; Seite 2, Zeilen 66-119; Seite 3, Zeilen 7-19; Abbildungen 3-8 *<br><br>-- | 1,2 |
| | GB - A - 1 529 831 (EMI LTD.) | 1 |

**KLASSIFIKATION DER ANMELDUNG (Int. Cl.³)**

A 61 B 6/02

**RECHERCHIERTE SACHGEBIETE (Int. Cl.⁴)**

A 61 B 6/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

./.

Der vorliegende Recherchenbericht wurde für alle Patentansprüche erstellt

| Recherchenort | Abschlußdatum der Recherche | Prüfer |
|---|---|---|
| Den Haag | 29-06-1981 | RIEB |

EPA form 1503.1   06.78

0037008

Europäisches
Patentamt

**EUROPÄISCHER RECHERCHENBERICHT**

Nummer der Anmeldung
EP 81 10 1991
-2-

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | * Seite 2, Zeilen 74-116; Abbildungen 2a,2b * | | |
| | -- | | |
| | US - A - 4 138 721 (D.P. BOYD/ BOARD OF TRUSTEES OF THE LELANE STANDARD JR. UNIVERSITY) | 1,3 | |
| | * Zusammenfassung; Spalte 4, Zeile 37 - Spalte 5, Zeile 37; Spalte 10,Zeilen 39-61; Spalte 11, Zeilen 1-50 und Abbildungen 1-4,16 * | | |
| | -- | | RECHERCHIERTE SACHGEBIETE (Int. Cl. 3) |
| | US - A - 4 086 492 (J.A. LODGE et al. / EMI LTD.) | 1 | |
| | * Zusammenfassung; Spalte 1, Zeilen 43-64; Spalte 4, Zeilen 36-61; Spalte 5, Zeilen 24-41 und Abbildung 4 * | | |
| | -- | | |
| P | EP - A - 0 024 028 (SIEMENS A.G.) | 1,3,4 | |
| | * Zusammenfassung; Seite 2, Zeile 31 - Seite 4, Zeile 21; Seite 5, Zeile 10 - Seite 6, Zeile 32 und Abbildungen 1-3 * | | |
| | ----- | | |

EPA Form 1503.2  06.78